# EUROPÄISCHE PATENTSCHRIFT

(11) **EP 1 530 641 B1**
(45) Veröffentlichungstag und Bekanntmachung des Hinweises auf die Patenterteilung: **24.01.2007**
(21) Anmeldenummer: 03787725.5
(22) Anmeldetag: 19.07.2003
(51) Int. Cl.: C12N 15/85

(54) **DNA-EXPRESSIONSKONSTRUKT ZUR MULTIPLEN GENEXPRESSION**
DNA EXPRESSION CONSTRUCT FOR MULTIPLE GENE EXPRESSION
PRODUIT RECOMBINE D'EXPRESSION D'ADN POUR L'EXPRESSION GENIQUE MULTIPLE

(30) Priorität: 19.07.2002 DE 10233812
(43) Veröffentlichungstag der Anmeldung: 18.05.2005
(73) Patentinhaber: Mologen AG, 14195 Berlin (DE)
(72) Erfinder: SMITH, Colin, PO Box 11-0236 Riad El SohlBeirut (LB); HUPPERTZ, Antje, 79541 Lörrach-Hauingen (DE); WITTIG, Burghardt, 14129 Berlin (DE)
(74) Vertreter: Omsels, Hermann-Josef
(86) Internationale Anmeldenummer: PCT/DE2003/002478
(87) Internationale Veröffentlichungsnummer: WO 2004/016786

(56) Entgegenhaltungen:
- WO-A-98/21322
- DE-A- 10 048 417
- US-A1- 2002 019 049
- SEEMAN NADRIAN C: "DNA nanotechnology: Novel DNA constructions" ANNUAL REVIEW OF BIOPHYSICS AND BIOMOLECULAR STRUCTURE, 1998, Seiten 225-248, XP009022846 Annual Reviews Inc. {a}, P.O. Box 10139, 4139 El Camino Way, Palo Alto, California 94306, USA Series: Annual Review of Biophysics and Biomolecular Structure (ISSN 1056-8700) ISBN: 0-8243-1827-7 in der Anmeldung erwähnt

## Beschreibung

Die Erfindung betrifft ein DNA-Expressionskonstrukt zur multiplen Genexpression, ferner ein Verfahren zu seiner Herstellung und ein Verbindungsmolekül (Linker) zur Verknüpfung von DNA-Expressionskassetten zur Herstellung eines DNA-Expressionskonstruktes zur multiplen Genexpression, sowie letztlich Verwendungsmöglichkeiten in der genetischen Vakzinierung.

Derartige Expressionskonstrukte sollen insbesondere auf dem Gebiet der genetischen Immunisierung, Onkotherapie und -prophylaxe eingesetzt werden.

Die Gentherapie steht vor einer Reihe noch unzureichend gelöster Probleme, wovon der effektive Transfer der therapeutisch wirksamen DNA in die Zielzellen mit der resultierenden Expression des transferierten Gens (Transfektion) ein bedeutendes ist.

Aufgrund der Vielzahl der Barrieren beim Transfer von genetischem Material in die Zelle (Plasmamembran, Endosom, Zellkern) ist die Transfektion von nackter DNA ein seltener und schlecht vorherzusagen Vorgang. So wird bei der Injektion von DNA in Gewebe ein Großteil der Zellen im Zielgewebe nicht transfiziert und erfolgreich transfizierte Zellen exprimieren die aufgenommene DNA nicht in gleicher Stärke.

Es ist eindeutig, dass diese Probleme bei der Notwendigkeit, sogar mehrere Gene in einer Zelle simultan zur Expression zu bringen, stark limitierend wirken, da sich die geringe Wahrscheinlichkeit, dass eine Zelle von einem Konstrukt transfiziert wird, für den Fall der Verwendung mehrerer unabhängiger Konstrukte negativ potenziert.

Oftmals ist jedoch für bestimmte gentherapeutische Ansätze eine kontrollierte, simultane Expression erforderlich. Dies gilt insbesondere für die Bereiche der Tumorbehandlung, der Vakzinierung und der Entwicklung von regulatorischen Systemen für die kontrollierte Genexpression. So konnte gezeigt werden, dass die kombinierte Expression von Zytokingenen mit dem Wachstumfaktor GM-CSF einen signifikanten antitumoralen Effekt auslöste, der in einem überraschenden Therapieerfolg mündete (Wittig et al., 2001, Hum Gene Ther 12: 267-278).

Es existieren eine Vielzahl von Methoden, welche die Koexpression mehrerer Gene in einer Zelle ermöglichen sollen. Die einfachste Methode ist die Kotransfektion zweier unabhängiger Expressionskonstrukte (im Folgenden auch: Vektoren). Eine andere Methode besteht in der Transfektion mit einem Vektor, der zwei unabhängige Expressionskassetten trägt.

Bei beiden Möglichkeiten besteht jedoch die Gefahr, dass die Transkripte sich signifikant bezüglich ihrer Anzahl, Prozessierung, Halbwertszeit und translationalen Effektivität und somit in der Menge des exprimierten Proteins unterscheiden, so dass ihr Einsatz durch Ineffektivität und schlechte Reproduzierbarkeit gekennzeichnet ist.

Virale und nicht virale Vektorsysteme, sog. multicistronische Vektoren, wurden entwickelt. Derartige Vektoren tragen die Information für zwei oder mehr Gene. Es konnte gezeigt werden, dass DNA-Vektoren, die für zwei verschiedene Antigene kodierten, eine stärkere Immunität induzierten, als es Vektoren, die nur für je ein Gen kodierten, vermochten (Morris et al., 2000, Vaccine 18: 2155-2163).

Die Konstruktion von di- und polycistronischen mRNAs durch die Verwendung von IRES (Internal Ribosome Entry Site) Elementen stellt eine weitere Möglichkeit zur Koexpression mehrerer Gene dar. Diese ermöglichen die Initiation der Translation durch Ribosomen unabhängig von der mRNA-cap-Struktur durch Sequenzelemente. Erstmalig wurden IRES-Elemente in der mRNA des Picornavirus entdeckt (Pelletier und Sonenberg, 1988, Nature 334: 320-325, Jang et al., 1988, Journal Virology 62: 2636-2643).

Für die Konstruktion bicistronischer Vektoren werden am häufigsten die IRES-Elemente des Poliovirus und des EMCV (Encephalomyocarditis Virus) verwendet (Dirks et al., 1993, Gene 128: 247-249).

Nachteilhafterweise variiert deren Effektivität stark in Abhängigkeit von der verwendeten Zelllinie (Borman et al., 1997, Nucleic Acids Res. 25: 925-932).

Die meisten der verfügbaren bicistronischen Expressionskassetten bestehen aus einem selektierbaren Marker oder einem Reportergen, welche auf der 3'-Seite des IRES Elementes und einer MCS (Multiple Cloning Site) für die Einfügung des gewünschten Gens angeordnet sind (Dirks et al., 1993, Gene 128: 247-249). Ebenso sind tri- und polycistronische Expressionssysteme unter Verwendung von IRES-Elementen bekannt (Zitvogel et al., 1994, Hum Gene Ther 5: 1493-1506, Fussenegger et al., 1998a, Nature Biotechnol. 16: 468-472, Mielke et al., 2000, Gene 254. 1-8).

Für die therapeutische Anwendung sind virale IRES-Elemente jedoch nur mit Vorbehalt einsetzbar. Die Rekombination viraler Sequenzen mit natürlich vorkommenden Viren stellt ein inhärentes Sicherheitsrisiko dar, da die Erzeugung neuer, pathogener Hybridviren befürchtet wird.

Multicistronische Vektoren können auch durch die Verknüpfung von Transkriptionseinheiten ohne IRES-Elemente konstruiert werden. Zum einen können die gewünschten Gene über verschiedene MCS in einen Plasmid-Vektor kloniert werden, und zum anderen können aus Plasmiden isolierte Expressionskassetten durch DNA-Linker zu linearen multicistronischen Vektoren verknüpft werden (Tsang et al., 1997, Bio Techniques 22: 68).

Untersuchungen der Expressionsraten linearer cis-verknüpfter Gene zeigten jedoch einen starken negativen Einfluss auf die Transkription derart angeordneter Expressionskassetten (Esperet et al., 2000, J Biol Chem 275: 25831-25839).

Plasmid-Vektoren für die Expression multipler Gene unterscheiden sich von den üblichen Plasmid-Vektoren durch die Anzahl der klonierten Transkriptionseinheiten. Dabei können Promotoren bzw. poly(A)-Sequenzen gleichen oder unterschiedlichen Ursprungs für die Transkriptionseinheiten verwendet werden.

Der Nachteil, der sich aus der Verwendung unterschiedlicher Promotoren ergibt, besteht darin, dass die Promotoren sich in ihrer Stärke unterscheiden und so die Expressionsraten der einzelnen Gene variieren können, wohingegen der Einsatz gleicher Promotoren zur Ausbildung von Sekundärstrukturen der DNA führen kann, welche einen Funktionsverlust der Promotoren zur Folge haben können.

Allen genannten Vektoren zur multiplen Genexpression ist gemeinsam, dass sie entweder auf Plasmiden oder aber viralen Vektoren basieren. Neben allen beschriebenen Unzulänglichkeiten der gegenwärtigen mehrfachkodierenden Vektoren weisen sie zusätzlich die Nachteile dieser Art von Vektoren auf. Die Nachteile viraler Vektoren, zu denen Instabilität des attenuierten Impfstammes zählen, und die Unzulänglichkeiten von Plasmid-DNA-Vektoren, wie die mit ihrer Verwendung einhergehende Verbreitung von Antibiotikaresistenzgenen (ausführlich beschrieben in EP 0 941 318 B1), sind hinlänglich bekannt.

Zusammenfassend sei hervorgehoben, dass die Effektivität einiger gentherapeutischer Behandlungsansätze durch die Expression multipler Gene signifikant verbessert werden kann. Es konnten jedoch trotz langjähriger Forschung keine mehrfachkodierenden Vektoren entwickelt werden, die effizient die gewünschten Gene exprimieren und ohne Sicherheitsbedenken in der Gentherapie oder genetischen Vakzinierung von Mensch und Tier eingesetzt werden können. Dies war bisher nur durch die Kotransfektion von zwei separaten Expressionskonstrukten möglich (Hum. Gene Ther. 2001 Feb.,10; 12(3): 267-78).

Eine Methode zur Verknüpfung von DNA-Fragmenten durch DNA-Verbindungselemente ist aus der Literatur bekannt. Seeman beschreibt DNA-Sequenzen zur Untersuchung von natürlich vorkommenden Verzweigungen, sog. Holliday-Junctions, die als topologische Sonderformen von B-DNA unter torsionalem Stress postuliert werden (Seeman: 1982, J Theor Biol 99: 237-247; Annu. Rev. Biophys. Biomol. Struct. 1998. 27:225-248). Die Anwendung dieser Strukturen blieb bisher auf kurze Oligonukleotid-Stücke zur Untersuchung von DNA-Strukturformen beschränkt.

Aus der DE 100 48 417 A1 ist eine polyfunktionale Verbindung bekannt, welche die Konjugation von zwei reaktiven funktionellen Gruppen mittels eines Linkers beschreibt. Als biologische Substanzen, welche derart gekoppelt werden können, werden auch Nukleinsäuren erwähnt, die Beispiele zeigen jedoch nur die Anwendungen der Erfindung zur Konjugation von Peptiden. Einen anderen Ansatz mehrere Gene zur effizienten Expression in Zellen einzuschleusen beschreibt die WO 01/87348 A2. Nukleinsäuren sollen durch kovalente Bindungen zu supramolekularen Strukturen, sog. Dendrimeren, verknüpft werden.

Ausgehend von diesem Stand der Technik ist es Aufgabe der Erfindung, ein für mehrere Gene kodierendendes DNA-Expressionskonstrukt zur Verfügung zu stellen, welches zur effizienten multiplen Genexpression führt und die Nachteile des Standes der Technik vermeidet. Ferner soll ein entsprechendes Verfahren zur Herstellung eines derartigen Expressionskonstruktes bereitgestellt werden.

Die vorliegende Erfindung löst dieses technische Problem durch die kennzeichnenden Merkmale der selbstständigen Ansprüche.

Danach ist erfindungsgemäß ein DNA-Expressionskonstrukt zur multiplen Genexpression vorgesehen,
- bestehend aus doppelsträngigen Bereichen, die wenigstens zwei Expressionskassetten enthalten, welche jeweils zumindest eine Promotorsequenz und eine kodierende Sequenz aufweisen,
- und wobei diese doppelsträngigen Bereiche über einen verzweigten Linker durch Holliday-Junctions miteinander verbunden sind,
- und die Expressionskassetten an den Enden, an denen sie nicht mit weiteren Expressionskassetten über DNA-Einzelstränge oder nichtkodierende DNA-Doppelstränge miteinander verbunden sind, gegen Exonukleaseabbau geschützt sind.

Ein solches für mehrere Gene kodierendes DNA-Expressionskonstrukt, das Träger mehrerer Expressionskassetten ist, kann Zellen gezielt transfizieren.

Unter Transfektion soll das Einbringen von Nukleinsäuresequenzen mittels biologischer, chemischer oder physikalischer Methoden in die Zelle verstanden werden, in dessen Folge es zu anhaltender oder vorübergehender Expression von durch diese Sequenzen kodierten Proteinen oder katalytisch wirksamen RNA-Transkripten in der transfizierten Zelle kommt.

Unter einem mehrere Gene kodierenden Vektor soll in diesem Zusammenhang ein DNA-Expressionskonstrukt verstanden werden, das aus mehreren miteinander verbundenen, aber unabhängigen Expressionskassetten besteht, wobei jede Expressionskassette aus einem Promotor, der kodierenden Sequenz und gegebenenfalls einer Terminationssequenz besteht. Zweckmäßigerweise wird zur Verwendung *in vivo* der DNA-Doppelstrang vor ubiquitär vorhandenen Exonukleasen geschützt, vorzugsweise durch Einbau von Thioat-modifizierten Nukleotiden, kovalentem Ringschluß mit haarnadelförmigen Oligodesoxynukleotiden (vgl. EP 0 941 318 B1) am unverknüpften Ende des Moleküls, oder anderen zum Stand der Technik gehörenden chemischen oder physikalischen Modifikationen, die geeignet sind, DNA-Moleküle vor enzymatischem Abbau zu schützen.

Unter multipler Genexpression wird im Sinne der Erfindung verstanden, dass eine Mehrzahl von Genen - gleichgültig ob es sich um identische oder unterschiedliche Gene handelt - von ein und demselben Genexpressionskonstrukt exprimiert werden.

Erfindungsgemäß ist auch ein Verbindungsmolekül (Linker) zur Verknüpfung von DNA-Expressionskassetten zur Herstellung eines DNA-Expressionskonstruktes zur multiplen Genexpression vorgesehen. Dieses wird hergestellt aus partiell zueinander komplementären einzelsträngigen Oligodesoxynukleotiden, deren Sequenzen so gewählt sind, dass sie untereinander zu einem oder mehreren nicht-kodierenden Doppelsträngen hybridisieren, wobei gegebenenfalls zwischen diesen Doppelsträngen einzelsträngige Bereiche verbleiben und darüber hinaus kohäsive Enden zur Hybridisierung von Expressionskassetten verbleiben.

Unter Linker sollen Oligonukleotide zur Verbindung von DNA-Expressionskassetten verstanden werden. Ein erfindungsgemäßer Linker besteht aus zwei oder mehr Strängen, wie an folgendem Beispiel für einen Linker, gebildet aus drei Oligonukleotiden, die mit drei 3 Expressionskassetten auf miteinander verbundenen Strängen A, B und C verbunden sind, illustriert werden soll: Das 5'-Ende von A hat zu dem 3'-Ende von B eine komplementäre Sequenz und bildet einen Doppelstrang, während das 3'-Ende von A mit dem 5'-Ende von C paart, und schließlich das 5'-Ende von B mit dem 3'-Ende von C paart. Diese Art von Verbindung von DNA-Doppelsträngen wird auch als Holliday-Junction bezeichnet (F.W. Stahl: Genetics. 1994 Oct;138(2):241-6), welche den Vorteil bietet, dass ausschließlich natürlich vorkommende Bausteine zum Verbinden der Expressionskassetten verwendet werden, was sich bei einer Verwendung der Erfindung zu medizinischen Zwecken aus toxikologischen Gründen als besonders vorteilhaft erweisen kann.

Die Erfindung sieht die Verwendung kovalent miteinander verbundener linearer Expressionskassetten vor, die lediglich aus einem die Transkription steuernden Initiator, vorzugsweise einem durch eine eukaryote DNA-Polymerase operablen Promoter wie z.B. dem CMV Promotor, und einer Terminatorsequenz, vorzugsweise einer Polyadenylierungssequenz, bestehen.

Die Darstellung von unverzweigten Expressionskonstrukten mit linearer, kovalent geschlossener Topologie ist in der EP 0 941 318 B1 gezeigt. Solche Expressionskonstrukte werden durch Ausschneiden mit einer Typ-II Restriktionsendonuklease aus einem dafür geeigneten Plasmid isoliert. Das entstandene kohäsive, also mit einem hybridisierbaren Einzelstrang-Überhang versehene Fragment, welches die Expressionskassette enthält, wird dann durch die Ligation von Haarnadelschleifen-Oligonukleotiden kovalent geschlossen. Das Plasmidrückgrat, welches nicht an der Ligationsreaktion mit den Oligonukleotiden teilnimmt und offenkettig bleibt, wird enzymatisch durch die Exonukleasefunktion der T7-DNA-Polymerase abgebaut und das entstandene linear-doppelsträngige, an den Enden kovalent geschlossene DNA-Polymer aufgereinigt. Durch diese Methode werden alle für die Plasmidherstellung benötigten Sequenzelemente aus dem Expressionskonstrukt entfernt. Solche sog. minimalistischen Genexpressionskonstrukte haben den Vorteil, dass sie beim Einsatz im Patienten nur noch Strukturen aufweisen, die für die therapeutische Wirkung essentiell sind, was letztlich die Nachteile von Plasmiden und auch der Genfähren viralen Ursprungs vermeidet.

Diese bekannte Methodik wird für die Herstellung der für mehrere Gene kodierenden Expressionskonstrukte erfindungsgemäß variiert. Danach ist ein Verfahren zur Herstellung eines DNA-Expressionskonstrukts zur multiplen Genexpression vorgesehen,
a) wobei partiell zueinander komplementäre einzelsträngige Oligodesoxynukleotide zu einem Verbindungsmolekül (Linker) hybridisiert werden,
b) wobei die Hybridisierung derart unvollständig erfolgt, dass daraus zwei oder mehr kohäsive Enden mit gleicher oder unterschiedlicher Basensequenz resultieren, und
c) wobei anschließend voneinander unabhängige Expressionskassetten aus linearen DNA-Doppelsträngen, welche zumindest aus einer Promotorsequenz und einer kodierenden Sequenz bestehen, durch komplementäre Überhänge mit den kohäsiven Enden des Verbindungsmoleküls (Linker) über Holliday-Junctions ligiert werden, und
d) wobei die endständigen doppelsträngigen Expressionskassetten gegen Exonukleaseabbau geschützt werden,
e) wobei das derart hergestellte DNA-Expressionskonstrukt gegebenenfalls durch Wiederholung der Verfahrensschritte a) bis c) um weitere verzweigte Verbindungsmoleküle (Linker) zur Konjugation von Expressionskassetten über Holliday-Junctions erweitert werden kann.

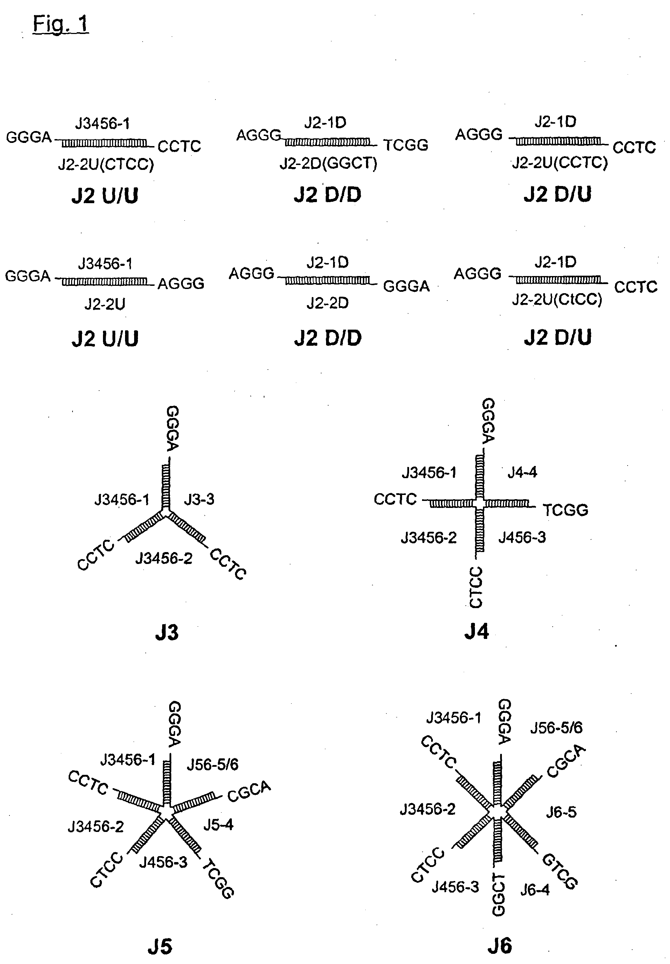

Wesentlicher Bestandteil des erfindungsgemäßen Expressionskonstrukts bzw. Vektors ist das Verbindungsmolekül (Linker). Dieses dient als Verzweigungsmolekül bzw. Trennungsmolekül der Expressionskassetten und ist aus diesem Grund selbst nichtkodierend. Die Anzahl der teilweise einzelsträngigen oder hybridisierenden Oligonukleotide korrespondiert mit der Anzahl der Verzweigungen (siehe Figur 1). Zur Erzeugung einer 3-fach-Verzweigung werden beispielsweise drei Oligonukleotide verwendet. Die für die DNA-Verzweigungen verwendeten Oligodesoxynukleotidsequenzen werden so gewählt, dass bei der Hybridisierung an den 5'-Enden der Oligonukleotide (beispielsweise) je ein vier Basen langes, überhängendes, kohäsives Ende erzeugt wird. Die Sequenzen der kohäsiven Enden können sich für jeden Arm einer Verzweigung unterscheiden, müssen es aber nicht. Die Expressionskassetten weisen ein zu dem kohäsisiven Ende komplementären Überhang auf. Hierdurch wird die gezielte Ligation der Expressionskassetten an die einzelnen "Arme" der Verzweigungen möglich.

Ein erfindungsgemäßes Expressionskonstrukt ist zur Verwendung in einem Verfahren zur Behandlung von Tumorerkrankungen vorgesehen.

Ein erfindungsgemäßes verzweigtes Verbindungsmolekül (Linker) zur Verknüpfung von wenigstens zwei DNA-Expressionskassetten zur Herstellung eines DNA-Expressionskonstruktes zur multiplen Genexpression besteht aus partiell zueinander komplementären einzelsträngigen Oligodesoxynukleotiden, wobei diese untereinander zu einem oder mehreren nicht-kodierenden Doppelsträngen mit kohäsiven Enden zur Hybridisierung von Expressionskassetten hybridisieren und die Doppelstränge über Holliday-Junctions miteinander verbunden sind.

Erfindungsgemäß ist zudem vorgesehen, einen Kit zur Verfügung zu stellen, der neben den üblichen Mitteln zur Hybridisierung und Ligation von Oligodesoxynukleotiden auch Oligodesoxynukleotide enthält, die zur Synthese von erfindungsgemäßen Verbindungsmolekülen geeignet sind, deren Sequenzen nämlich so gewählt sind, dass sie zu verzweigten Linkern mit einem oder mehreren nicht-kodierenden Doppelsträngen mit kohäsiven Enden zur Hybridisierung von Expressionskassetten hybridisieren und die Doppelstränge über Holliday-Junctions miteinander verbunden sind. Abhängig von den verwendeten Oligodesoxynukleotiden, kann der Anwender so Verbindungsmoleküle mit der von ihm benötigten Anzahl von Enden zur Ligation von Expressionskassetten herstellen.

Zum Schutz vor Exonukleaseabbau werden die Transkriptionseinheiten nur auf einer Seite mit einem Haarnadelschleifen-Oligonukleotid kovalent geschlossen, die andere Seite gezielt mit einem Ende des kohäsiven DNA-Verbindungsmoleküls ligiert. Auf diese Weise können vorteilhafterweise die Expressionskassetten beliebiger Gene miteinander kombiniert und in gezielter Orientierung zueinander verknüpft werden.

Es ist erfindungsgemäß daher vorgesehen, dass der Schutz vor Exonukleaseabbau durch einen kurzen Einzelstrangbereich aus drei bis acht Desoxynukleotiden erreicht wird, der die beiden Stränge des linearen Doppelstranges kovalent miteinander verknüpft. Zur effektiven Transfektion der Zellen kann in dem kurzen Einzelstrangbereich das DNA-Expressionskonstrukt mit einem oder mehreren Peptiden kovalent verknüpft sein. Dies erfolgt dadurch, dass die haarnadelförmigen Schleifen aus Nukleotiden mit einem oder mehreren Peptiden an einem oder mehreren kovalent modifizierten Nukleosidresten der Nukleotide der Haarnadelschleife kovalent verknüpft sind. Vorzugsweise besteht dabei das Peptid aus zwischen 3 und 30 Aminosäuren, von denen mindestens die Hälfte basische Aminosäuren aus der Gruppe Arginin und Lysin sind. Als vorteilhaft hat sich für das Peptid die Aminosäuresequenz PKKKRKV (Polin-Lysin-Lysin-Lysin-Arginin-Lysin-Valin) erwiesen.

Die Erfindung betrifft somit ein DNA-Expressionskonstrukt zum Transport von genetischer Information, bestehend aus doppelsträngigen DNA-Expressionskassetten, verbunden durch ein Verbindungsmolekül, wobei ein jedes Verbindungsmolekül (Linker) aus partiell zueinander komplementären einsträngigen Oligodesoxynukleotiden besteht, deren Sequenzen so aufeinander abgestimmt sind, dass sie untereinander derart zu einem oder mehreren Doppelsträngen hybridisieren, dass an den jeweiligen Enden der einsträngigen Oligodesoxynukleotide auf beiden Seiten des Linkers kohäsive Enden erzeugt werden, zu denen Sequenzabschnitte der Expressionskassetten komplementär sind. Die Erfindung betrifft somit ein mehrere Gene kodierendes Vektorsystem, das auf der Verknüpfung unabhängiger Expressionskassetten durch einen (zentralen) Linker beruht. Erfindungsgemäß können beliebig viele Expressionskassetten miteinander verknüpft werden.

Bezüglich der vorliegenden Erfindung ist weder die Kotransfektion von zwei separaten Vektoren noch die in der DE 100 48 417 A1 beschriebene Methode, die kodierenden Gensequenzen zusammen mit den notwendigen Steuerelementen (Promotor, poly(A)-Signal) chemisch miteinander zu verbinden, ein naheliegender Stand der Technik. Zwar werden bei der Kotransfektion mittels Lipofektion ebenfalls unterschiedliche Vektoren in räumliche Nähe zueinander gebracht und in die Zelle eingeschleust, jedoch ist das zugrundeliegende Prinzip dieser Methode ein anderes. Zudem entstehen nachteilhafterweise durch die beschriebene chemische Konjugation von Expressionskonstrukten Strukturen mit einem deutlich größeren Molekulargewicht, welche sich bekanntermaßen schwieriger in Zellen einschleusen lassen. Das gleiche trifft auch auf das in der WO 01/87348 A2 beschriebene Verfahren zum Aufbau eines Nukleinsäuren enthaltenden Dendrimers zu.. Zusätzlich ist das dargestellte Verfahren zum Aufbau von Dendrimeren ein mehrstufiger und aufwendiger Herstellungsprozess.

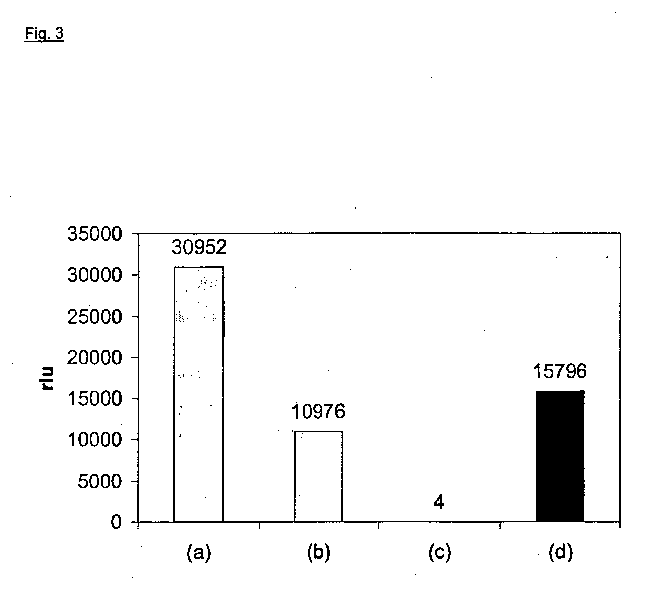

Zum Nachweis der Koexpression mehrerer Expressionskassetten eines erfindungsgemäßen Expressionskonstruktes diente ein drei Gene kodierender Vektor, der für die Firefly-, die Renilla-Luciferase und das grün fluoreszierende Protein (GFP) kodierte. Der Expressionsnachweis erfolgte *in vitro* in Säugerzellen (s. Fig. 3). Alle drei Proteine konnten detektiert werden. Ebenso konnte die simultane Expression der Proteine durch diese Vektoren in derselben Zelle nachgewiesen werden. Das ist von Vorteil, da beispielsweise ein Ansatz für die Immunotherapie von Tumorerkrankungen darauf basiert, mehrere Zytokin-Gene zusammen mit anderen kostimulatorischen Faktoren als DNA-Vakzine einzusetzen (WO 02/060476 A2). Bei diesen erfolgversprechenden Therapieansätzen ist es bedeutsam, dass die exprimierten Zytokine und kostimulatorischen Faktoren in möglichst enger räumlicher Nähe zueinander, bei vielen Anwendungen in derselben Zelle, in den Effektororganen des Immunsystems exprimiert werden, da kostimulatorische Signale nur in Zusammenhang mit Antigenen eine wirksame Verstärkung der Impfung liefern.

Bei dem Expressionsnachweis des drei Gene kodierenden Konstrukts *in vivo* mit äquivalenten Mixturen aus Firefly-, Renilla-Luciferase und eGFP Plasmiden sowie mit für diese Proteine jeweils einzeln kodierenden Vektoren konnte überraschend gezeigt werden, dass die Expressionsraten für die drei Gene kodierenden erfindungsgemäßen Konstrukte höher waren, als für die für die einzelnen Gene kodierenden Vektoren (s. Fig. 3).

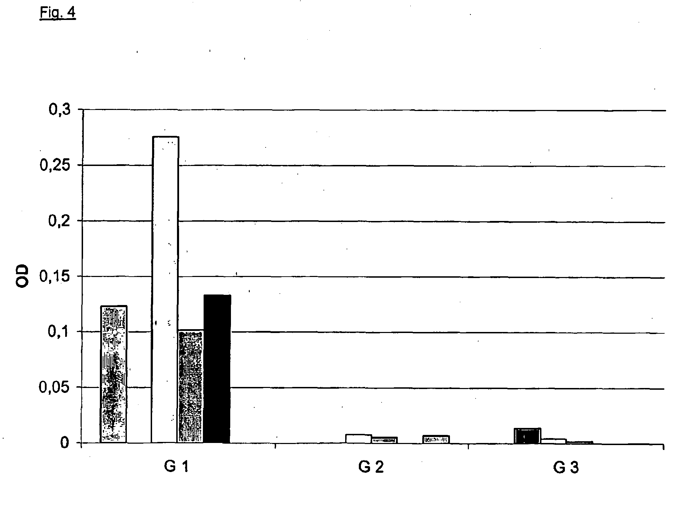

Das erfindungsgemäße DNA-Expressionskonstrukts kann zum Transport genetischer Information entsprechend einem oder mehreren der vorhergehenden Ansprüche als Bestandteil eines Arzneimittels zur genetischen Vakzinierung verwendet werden (s. Fig. 4).

Weitere Anwendungsgebiete der erfindungsgemäßen Konstrukte liegen unter anderem in der Expression multipler Untereinheiten heteromultimerer Proteine (Immunglobuline, Zellrezeptoren, Interleukine, Enzyme, Transkriptionsfaktoren), sowie in der Expression verschiedener heterologer Proteine mit dem Ziel, unterschiedliche, kombinierte oder synergistische onkolytische oder auch toxische Effekte zu induzieren.

Die erfindungsgemäßen mehrere Gene kodierenden Vektoren haben den Vorteil, dass sie zur multiplen Genexpression führen, wobei die Kombination der Transgene beliebig verändert werden kann und so auf die Anforderungen spezifischer Anwendungen eingegangen werden kann. Daneben besitzen diese Art von Vektoren keine Markergene oder kodierende Sequenz viralen Ursprungs und bestehen nur aus Sequenzen, die direkt für die Expression der therapeutischen Gene notwendig sind.

Im Unterschied zum Stand der Technik ist für die erfindungsgemäßen Vektoren die Komplexität der benötigten zellulären Faktoren geringer als für Vektoren, die zusätzlich zu dem Promotor IRES Elemente enthalten, deren Aktivität aus diesem Grund sehr stark variieren kann.

Weitere vorteilhafte Methoden zur Anwendung der Erfindung sind die biologischen Transfektionsmethoden wie der rezeptorvermittelte Gentransfer. Hierbei beispielsweise werden die mehrere Gene kodierenden DNA-Expressionssysteme kovalent mit einem oder mehreren Peptiden verbunden.

Weitere vorteilhafte Ausgestaltungsformen der Erfindung ergeben sich aus den Unteransprüchen, der Beschreibung und den Figuren. Die überraschende Wirkung des erfindungsgemäßen mehrere Gene kodierenden DNA-Expressionskonstruktes, sowie das erfindungsgemäße Verfahren wird anhand der Figuren und den Ausführungsbeispielen deutlich; dabei bedeuten:

| | |
|---|---|
| pMOK-Luc | Plasmid, kodierend für Firefly-Luciferase |
| | |
| pMOK-eGFP | Plasmid, kodierend für enhanced Green Flourescent protein |
| | |
| pMOK-Ren | Plasmid, kodierend für Renilla-Luciferase |
| | |
| MOK-Luc-M | Lineares kovalent geschlossenes Expressionskonstrukt, kodierend für Firefly-Luciferase |
| | |
| MOK-eGFP-M | Lineares kovalent geschlossenes Expressionskonstrukt, kodierend für enhanced Green Flourescent protein (GFP) |
| | |
| MOK-Ren-M | Lineares kovalent geschlossenes Expressionskonstrukt, kodierend für Renilla-Luciferase |
| | |
| pMOK-h B7.1 | Kontrollplasmid, kodierend für das CD 80-Gen (B7.1) als Negativkontrolle |
| | |
| MOK-Luc U/eGFP U/Ren U-M | drei erfindungsgemäß verknüpfte lineare kovalent geschlossene Expressionskassetten (J3, s. Fig. 1), kodierend für Firefly-Luciferase, enhanced Green Flourescent Protein (eGFP) und Renilla-Luciferase |

Es zeigt:
- **Fig. 1:**: Schematische Darstellung der Strukturen von erfindungsgemäßen DNA-Verbindungsmolekülen (Linkern) am Beispiel von Linkern mit 2, 3, 4, 5 und 6 Funktionalitäten zur Verbindung mit Expressionskassetten (J2, J3, J4, J5 und J6; wobei "J" für "Junction" steht und die Zahl für "Anzahl der Verbindungsstellen mit Expressionskassetten" sowie "D" und "U" die O-rientierung der Expressionskassetten angibt (U=Upstram; D=Downstream)). Die Sequenzen der verwendeten Oligodesoxynukleotide sind in der Tabelle 1 angegeben. In Figur 1 bedeutet:
(a) J2 die Verknüpfung zweier unabhängiger Expressionskassetten an deren "upstream"-Enden in divergenter Anordnung (J2 U/U), an deren "downstream"-Enden in konvergenter Anordnung (J2 D/D) und an deren upstream und downstream Enden in der Tandem Anordnung (J2 D/U)
(b) J2 für die Verknüpfung zwei gleicher Expressionskassetten an deren upstream Enden in divergenter Anordnung (J2 U/U), an deren downstream Enden in konvergenter Anordnung (J2 D/D) und an deren upstream und downstream Enden in der Tandem Anordnung (J2 D/U)
(c) J3, J4, J5 und J6 die Verknüpfung unterschiedlicher Expressionskassetten an deren kohäsiven Enden.

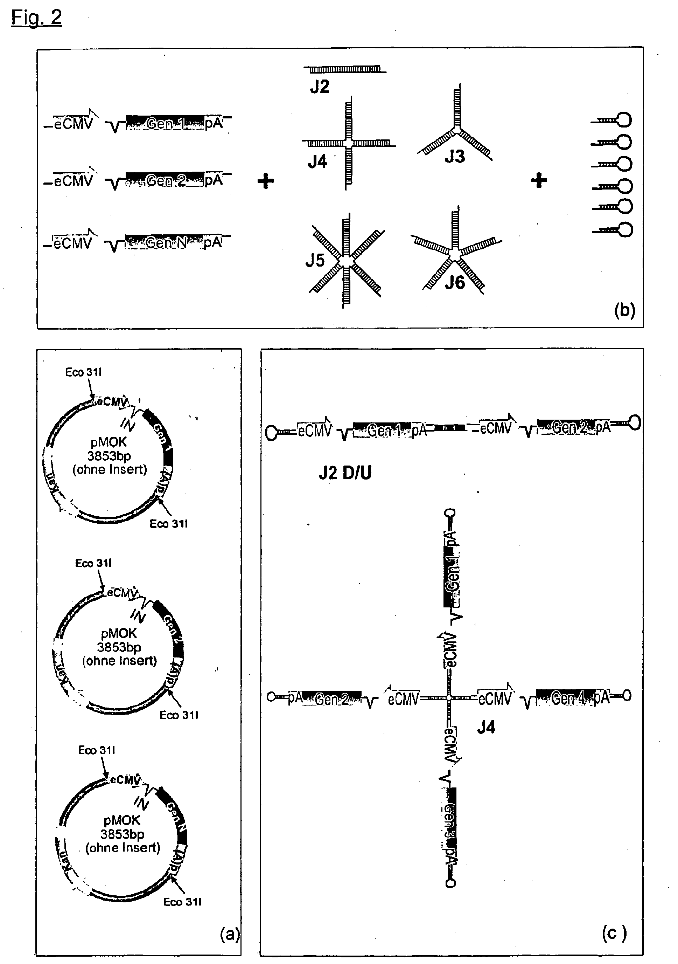

- **Fig.2:**: Die Komponenten der Ligationsreaktion zur Bildung eines erfindungsgemäßen Vektors. Fig. 2 zeigt:
(a) Vektoren zur Vermehrung der Expressionskassetten
(b) Bestandteile eines erfindungsgemäßen DNA-Expressionskonstruktes: ausgeschnittene lineare Expressionskassetten, Verbindungsmolekül (Linker) sowie Haarnadelschleifen zum Schutz gegen Exonukleaseabbau der offenen Enden der Expressionskasstten
(c) den schematischen Aufbau eines 4 Gene kodierenden erfindungsgemäßen Expressionskonstruktes
- **Fig. 3:**: Expressionsvergleich von einfach- und mehrfachkodierenden Vektoren für Firefly- und Renilla-Luciferase sowie eGFP. Die Expression wurde anhand der relativen Lichteinheiten (rlu) bestimmt nach Transfektion von HeLa-Zellen mit:
(a) mit einer Mischung aus unabhängigen Plasmiden pMOK-Luc, pMokeGFP, pMOK-Ren
(b) mit einer Mischung aus unabhängigen linearen kovalent geschlossenen Expressionskonstrukten MOK-Luc-M, MOK-eGFPM, MOK-Ren-M
(c) mit dem Kontrollplasmid pMOK-hb 7.1
(d) mit einem für alle drei Gene kodierenden erfindungsgemäßen MOK-Luc U/eGFP U/Ren U-M Vektor.
- **Fig.4:**: Die ELISA-Bestimmung des Gesamt IgG-Antikörpertiters durch Messung der Optischen Dichte (OD) gegen das Hepatitis B Antigen in Woche 4 nach der Immunisierung mit erfindungsgemäßen dreifach kodierenden Vektoren. Die einzelnen Säulen stellen individuelle Tiere dar.
G 1: Vektor, kodierend für HBsAg, CD40L und mB7.1
G 2: Vektor, kodierend für Luciferase, CD40L und mB7.1 als Negativkontrolle für spezifische Antikörper gegen HBsAg
G 3: Vektor, kodierend für HBsAg, Luciferase und β-Galactosidase als Negativkontrolle für immunstimulatorische Effekte der Moleküle CD40L und mb7.1.

### Herstellung mehrere Gene kodierender Vektoren

### Annealing der Linker J2, J3, J4, J5 und J6

Die LinkerJ2, J3, J4, J5 und J6 setzen sich aus zwei, drei, vier, fünf bzw. sechs einsträngigen Oligodesoxynukleotiden (ODNs) zusammen, welche in ihrer Sequenz zueinander komplementäre Abschnitte besitzen. So besteht der Linker J2 U/U (Upstream/Upstream) aus den 36mer ODNs J3456-1 und J2-2U, wobei von den je 36 Nukleotiden nur 32 zueinander komplementär sind. Dadurch werden bei der Hybridisierung von J3456-1 und J2-2U auf beiden Seiten des Linkers kohäsive Enden erzeugt, zu denen die Upstream-Überhänge der Expressionskassetten komplementär sind (s. Fig. 1). Der Linker erhält durch diese Hybridisierung quasi eine neue "funktionelle Reaktionsstelle", die ihn befähigt, weitere Hybridisierungen einzugehen (im Sinne der Erfindung also mit einer Expressionskassette). Die Hybridisierung könnte man als bewusst unvollständig bezeichnen oder aber als Ergebnis einer gezielten Hybridisierung zur Schaffung weiterer potentieller Hybridisierungsstellen.

Ähnlich verhält es sich mit den Linkern J3, J4, J5 und J6 (s. Fig. 1). Hierbei sind nur jeweils 16 Basen zweier ODNs zueinander komplementär, so dass jedes ODN mit je zwei ODNs hybridisieren kann. Die so entstandenen sternförmigen Strukturen besitzen an den 5'-Enden je vier Basen lange kohäsive Enden. Jeder der Überhänge enthält eine andere spezifische Basenfolge. Dabei wurden für alle Verzweigungen die Sequenzen in der gleichen Reihenfolge verwendet, d.h. dass z.B. die Sequenz des kohäsiven Endes am ersten Arm für J3, J4, J5 und J6 übereinstimmt. Hierdurch wird die gezielte Ligation der Expressionskassetten an die einzelnen Arme der Verzweigungen ermöglicht (s. Fig. 1).

Die ODNs hybridisierten während des langsamen Abkühlens, nachdem sie in wässriger Lösung für 3 Minuten auf 90°C erhitzt wurden.

**Tabelle1: Sequenzen der verwendeten Oligonukleotide (TIB-Molbiol, Berlin). Die aus verschiedenen Kombinationen der Oligonukleotide entstehenden Verzweigungen von DNA-Doppelsträngen sind in den Figuren 1 - 3 illustriert.**

| **Bezeichnung** | **Sequenz (5' → 3')** |
|---|---|
| J3456-1 | 5'-P-ggg agg ttc agc cgc aat cct cgc ctg cac tct acc-3' |
| | |
| J3456-2 | 5'-P-cct cgg tag agt gca ggc gat gag cac gag tct tgc-3' |
| | |
| J3-3 | 5'-P-ctc cgc aag act cgt gct cag gat tgc ggc tga acc-3' |
| | |
| J456-3 | 5'-P-ctc cgc aag act cgt gct cac cga atg cca cca cgc-3' |
| | |
| J4-4 | 5'-P-ggc tgc gtg gtg gca ttc ggg gat tgc ggc tga acc-3' |
| | |
| J5-4 | 5'-P-ggc tgc gtg gtg gca ttc gga cta tgg cac aca tcc-3' |
| | |
| J6-4 | 5'-P-ggc tgc gtg gtg gca ttc ggc gtc cag ctc tga tcc-3 |
| | |
| J6-5 | 5'-P-gtc ggg atc aga gct gga cga cta tgg cac aca tcc-3' |
| | |
| J56-5/6 | 5'-P-acg cgg atg tgt gcc ata gtg gat tgc ggc tga acc-3' |
| | |
| J2-2 | 5'-P-cct cgg tag agt gca ggc gag gat tgc ggc tga acc-3' |
| | |
| J2-1 D | S-P-agg ggg ttc agc cgc aat cct cgc ctg cac tct acc-3' |
| | |
| J2-2D | 5'-P-agg ggg tag agt gca ggc gag gat tgc ggc tga acc-3' |
| | |
| J2-2 U | 5'-P-ggg agg tag agt gca ggc gag gat tgc ggc tga acc-3' |
| | |
| J2-2 U (CTCC) | 5'-P-ctc cgg tag agt gca ggc gag |
| | gat tgc ggc tga acc-3' |
| | |
| J2-2 D (GGCT) | 5'-P-ggc tgg tag agt gca ggc gag gat tgc ggc tga acc-3' |
| | |
| J3456-2/ggga | 5'-P-ggg agg tag agt gca ggc gat gag cac gag tct tgc-3' |
| | |
| J456-3/ggga | 5'-P-ggg agc aag act cgt gct cac cga atg cca cca cgc-3' |

### Klonierung neuer Eco31I Überhänge in pMOK mittels PCR

Das Grundprinzip der Herstellung der mehrere Gene kodierenden Vektoren beruht auf der Ligation des gewünschten Linkers (J2, J3, J4, J5 oder J6) mit mehreren linearen Expressionskassetten. Die entsprechenden Expressionskassetten werden aus dem Plasmid pMOK an den Eco31I Schnittstellen ausgeschnitten. Über die dabei entstehenden 4 Basen-Überhänge können die Expressionskassetten an den komplementären 4 Basen-Überhang des Linkers ligiert werden.

Da die Ligation der Expressionskassetten an die einzelnen Arme der Linker spezifisch erfolgen soll, müssen zu den zwei bestehenden Sequenzen der Eco31I Schnittstellen in pMOK (upstream Überhang: 5'TCCC 3', downstream Überhang: 5'CCCT 3') neue Eco31I Schnittstellensequenzen kloniert werden.

Die Klonierung erfolgt mittels Polymerase-Ketten-Reaktion (PCR), bei der die Sequenzen der PCR Primer so gewählt werden, dass sie jeweils im Bereich der upstream bzw. downstream Eco31I Schnittstelle mit 21 Basen des pMOK-Plasmides hybridisieren können. Der zum Bereich der upstream Eco31I Schnittstelle komplementäre PCR Primer enthält im Bereich der Schnittstelle die neue gewünschte Sequenz. Die 3'-Enden der Primer sind so aufeinander gerichtet, dass das pMOK Plasmidrückgrat amplifiziert werden kann.

Die Herstellung der mehrfachkodierenden Vektoren beruht auf der Ligation unabhängiger Transkriptionseinheiten mit doppelsträngigen verzweigten DNA Linkern durch T4-DNA-Ligase.

### Herstellung der Expressionskassetten

Plasmide vom pMOK-Typ, welche die entsprechenden Gensequenzen tragen, werden mit dem, Restriktionsenzym Eco31 I über Nacht bei 37°C vollständig verdaut (s. Fig. 2). Durch den Restriktionsverdau werden je Plasmid je zwei DNA-Fragmente erzeugt. Das eine Fragment besteht aus dem Kanamycin-Resistenzgen, sowie anderen zur Propagierung des Plasmides in Bakterien notwendigen Sequenzen. Das andere Fragment besteht aus den Sequenzen, die Bestandteil der Expressionskassette werden sollten: enhanced CMV-Promotor, chimäres Intron, der entsprechenden Gensequenz und der Polyadenylierungssequenz aus SV-40.

Das stöchiometrische Verhältnis *Linker* : *Expressionskassette* : *Haarnadelschleifen-ODN* betrug 1:2:10. Hierbei wird das Verhältnis der Expressionskassette und des Haarnadelschleifen-ODN zum Linker auf die Anzahl der Überhänge bezogen (s. Fig. 2). Je nach benötigter Produktmenge wird die Reaktion in unterschiedlichen Volumina durchgeführt, wobei der Linker in einer molaren Konzentration von 0,025 µM vorlag, und ATP in einer molaren Endkonzentration von 0,1 mM. Die Konzentration der T4-DNA-Ligase im Ansatz richtete sich nach der Plasmid-DNA-Menge. Pro µg DNA werden 0,01 Einheiten Ligase verwendet. Um die Expressionskassetten aus den Plasmiden zu schneiden und die Rückligationsrate so klein wie möglich zu halten, wird gleichzeitig 5 U/µg DNA Eco31I zugegeben. Die Inkubation der Reaktion erfolgt über Nacht bei 37 °C. Die schematische Darstellung der in den Prozess eingehenden Komponenten ist in Figur 2 dargestellt, wobei beachtet werden muss, dass pro Ligationsreaktion nur ein Typ Verbindungsmolekül verwendet wird.

Alle nicht vollständig geschlossenen DNA-Stränge, Restvektor und ODNs können nach Abschluß der Ligationsreaktion durch Zugabe von T7-Polymerase abgebaut werden.

### Verwendete Säugerzelllinien

K562 Zellen (Erythroleukämiezelllinie, DSMZ GmbH, Braunschweig) und HeLa Zellen (menschliches Cervixcarcinom, ATCC, USA) wurden kultiviert.

### Transfektion der Zellen mittels Elektroporation

K562-Zellen wurden mit Firefly- und/oder Renilla-Luciferase kodierenden Vektoren und HeLa-Zellen mit den für eGFP und DsRed (Red Flourescent Protein aus Discosoma) kodierenden Vektoren elektroporiert.

Vor dem Elektroporieren wurden die K562 Zellen abzentrifugiert, in ein kleines Volumen frisches RPMI Medium aufgenommen und die Zellzahl bestimmt. Das Supensionsvolumen wurde durch erneute Zugabe von frischem Medium so eingestellt, dass die Zellkonzentration 1x10⁷ Zellen/ml betrug. Davon wurden zu jeder DNA-Probe (1 µg) 500 µl gegeben, gemixt und in eine Elektroporationsküvette (Eurogentec, Belgien) gefüllt. Die Elektroporation erfolgte bei 300 V, 1050 µF und 99 Ω. Anschließend wurde die Zellsuspension sofort in 10 ml vorgewärmtes RPMI Medium überführt und über Nacht im Brutschrank inkubiert.

HeLa-Zellen wurden ebenso behandelt. Nach der Trypsinierung wurden sie in DMEM Medium (Gibco BRL, Berlin) aufgenommen und die Zellzahl bestimmt. Zum Einstellen der Zellkonzentration auf 1x10⁷ Zellen/ml wurden die Zellen abzentrifugiert, der Überstand verworfen und die Zellen im gewünschten Volumen DMEM-Medium resupendiert. Weiter wurde wie für die K562 Zellen beschrieben verfahren.

### Lipofektion der Zellen

Die Transfektion der Zellen erfolgt mit Hilfe kationischer Lipide. Lipofectin^{®} (Invitrogen, Karlsruhe) wurde für die Transfektion von HeLA-Zellen mit den dreifachkodierenden MOK-Luc U/eGFP U/Ren U-M und MOK-eCFP (enhanced Cyan Flourescent Protein) U/eGFP U/DsRed U-M Vektoren eingesetzt. Um die Transfektionseffizienz zu erhöhen, wurde für die Lipofektion der MOK-eCFP U/eGFP U/DsRed U-M Vektoren zusätzlich das PLUS^{™} Reagenz (Invitrogen, Karlsruhe) verwendet.

Am Tag vor der Transfektion wurden in 6-Loch-Platten pro Loch 1.6 x 10⁵ Zellen für die Lipofektion der MOK-Luc U/eGFP U/Ren U-M-Konstrukte und 2.5 x 10⁵ Zellen für die Lipofektion der MOK-eCFP U/eGFP U/DsRed U-M ausgesät.

Die Inkubation der Proben, die ohne den Zusatz des PLUS^{™} Reagenz transfiziert wurden, erfolgte für 45 Minuten (RT) in 100 µl serumfreiem DMEM Medium. Gleichzeitig wurde Lipofectin® im Verhältnis 1:3 zur DNA Menge in 100 µl serumfreien DMEM aufgenommen. Anschließend wurde der Lipofectin®/DMEM Mix zu den Proben gegeben und 10 Minuten bei RT inkubiert. Das Medium wurde aus den 6-Loch-Platten entfernt und die komplexierte DNA auf die Zellen gegeben. Nach 4 h wurde die Transfektionslösung entfernt und durch frisches Medium ersetzt.

Die Auswertung der Versuche erfolgte nach 16 - 24 h am Fluoreszenzmikroskop und durch den Dual-Luciferase Assay.

### Luciferase Assay

Die Transfektionsversuche wurden durch die Detektion der Markerproteine Renilla-Luciferase und Firefly-Luciferase mittels eines Dual®Luciferase Reporter Assays (Promega, Mannheim) ausgewertet.

### Antikörperbestimmung nach Immunisierung mit HBsAg

Um zu überprüfen, ob die erfindungsgemäßen Vektoren auch zu einer Gen- Expression im lebenden Organismus fähig sind, wurden *in vivo* Versuche an Mäusen mit für das Hepatitis-B-Öberflächenantigen HBsAg (Subtyp ay) kodierenden Vektoren durchgeführt (s. Fig. 4). Um die *in vivo* Funktionsweise des für mehrere Gene kodierenden Vektorsystems zu bestimmen, wurde ein dreifachkodierender Vektor eingesetzt. Dieser bestand aus den Expressionskassetten für das Hepatitis B Antigen und der beiden kostimulatorischen Moleküle CD40L und mB7.1, die die spezifische Immunantwort modulieren und verstärken sollten (Gruppe 1=G 1). Als Negativkontrolle für die Antigen Expression wurde das Reportergen Luciferase neben den beiden kostimulatorischen Molekülen eingesetzt (Gruppe 2 = G 2). Als Negativkontrolle für CD40L und mB7.1 wurden diese in Gruppe 3 (G 3) durch die Reportergene Luciferase und β-Galaktosidase ersetzt. Der Nachweis der Expression erfolgte durch die Antikörperbestimmung gegen das Hepatitis B Antigen mittels ELISA. Die verschiedenen dreifachkodierenden Vektoren wurden in Immunisierungsversuchen in drei Gruppen, jede bestehend aus 6 BALB/c Mäusen, eingesetzt. Äquimolare DNA Konzentrationen wurden in 1 M Natriumhydrogenphosphatpuffer gelöst und intradermal in die Tiere injiziert. Nach Woche 1 bis 5 wurde aus den Seren der Mäuse der Gesamt IgG-Gehalt bestimmt. Die Ergebnisse aus Woche 4 sind in Fig. 4 dargestellt. Die drei Gruppen, bestehend aus je 6 Mäusen, wurden mit folgenden erfindungsgemäßen dreifachkodierenden Vektoren immunisiert:

| | |
|---|---|
| Gruppe 1 (G 1): | erfindungsgemäßer Vektor, kodierend für HBsAg, CD40L und mB7.1 |
| | |
| Gruppe 2 (G 2): | erfindungsgemäßer Vektor, kodierend für Luciferase, CD40L und mB7.1 als Negativkontrolle für spezifische Antikörper gegen HBsAg |
| | |
| Gruppe 3 (G 3): | erfindungsgemäßer Vektor, kodierend für HBsAg, Luciferase und β-Galactosidase als Negativkontrolle für immunstimulatorische Effekte der Moleküle CD40L und mb7.1. |

Die Säulen in Fig. 4 stellen jeweils individuelle Tiere da. In allen drei Gruppen sind die erfindungsgemäßen Vektoren in der Lage, auch *in vivo* zur Genexpression zu führen. Dabei zeigt die Gruppe 1 den mit Abstand höchsten Antikörpertiter. Der Vergleich mit Gruppe 3, die das HBsAg ohne die kostimulatorischen Gene für CD40L und mB7.1 verabreicht bekamen, zeigt den Vorteil der Dreierkombination bei der Impfung gegen HBsAg durch den dreifachkodierenden Vektor.

## Patentansprüche

1. DNA-Expressionskonstrukt zur multiplen Genexpression,
- bestehend aus doppelsträngigen Bereichen, die wenigstens zwei Expressionskassetten enthalten, welche jeweils zumindest eine Promotorsequenz und eine kodierende Sequenz aufweisen,
- und wobei diese doppelsträngigen Bereiche über einen verzweigten Linker durch Holliday-Junctions miteinander verbunden sind,
- und die Expressionskassetten an den Enden, an denen sie nicht mit weiteren Expressionskassetten über DNA-Einzelstränge oder nichtkodierende DNA-Doppelstränge miteinander verbunden sind, gegen Exonukleaseabbau geschützt sind.

2. DNA-Expressionskonstrukt zur multiplen Genexpression nach Anspruch 1, wobei die Expressionskassetten für identische oder unterschiedliche Gene kodieren.

3. DNA-Expressionskonstrukt zur multiplen Genexpression nach Anspruch 1, wobei der Schutz vor Exonukleaseabbau durch einen kurzen Einzelstrangbereich aus drei bis acht Desoxynukleotiden erreicht wird, der die beiden Stränge des linearen Doppelstranges kovalent miteinander verknüpft.

4. DNA-Expressionskonstrukt zur multiplen Genexpression nach Anspruch 3, wobei dieses in dem kurzen Einzelstrangbereich mit einem oder mehreren Peptiden kovalent verknüpft ist.

5. DNA-Expressionskonstrukt zur multiplen Genexpression nach Anspruch 4, wobei das Peptid aus zwischen 3 und 30 Aminosäuren besteht, von denen mindestens die Hälfte basische Aminosäuren aus der Gruppe Arginin und Lysin sind.

6. DNA-Expressionskonstrukt zur multiplen Genexpression nach Anspruch 4 oder 5, wobei das Peptid die Aminosäuresequenz PKKKRKV (Polin-Lysin-Lysin-Lysin-Arginin-Lysin-Valin) aufweist.

7. Verfahren zur Herstellung eines DNA-Expressionskonstrukts nach einem der Ansprüche 1 bis 6,
a) wobei partiell zueinander komplementäre einzelsträngige Oligodesoxynukleotide zu einem verzweigten Verbindungsmolekül (Linker) hybridisiert werden,
b) wobei die Hybridisierung derart unvollständig erfolgt, dass daraus zwei oder mehr kohäsive Enden mit gleicher oder unterschiedlicher Basensequenz resultieren, und
c) wobei anschließend voneinander unabhängige Expressionskassetten aus linearen DNA-Doppelsträngen, welche zumindest aus einer Promotorsequenz und einer kodierenden Sequenz bestehen, durch komplementäre Überhänge mit den kohäsiven Enden des Verbindungsmoleküls (Linker) über Holliday-Junctions ligiert werden, und
d) wobei die endständigen doppelsträngigen Expressionskassetten gegen Exonukleaseabbau geschützt werden,
e) wobei das derart hergestellte DNA-Expressionskonstrukt gegebenenfalls durch Wiederholung der Verfahrensschritte a) bis c) um weitere verzweigte Verbindungsmoleküle (Linker) zur Konjugation von Expressionskassetten über Holliday-Junctions erweitert werden kann.

8. Verfahren nach Anspruch 7, wobei das verzweigte Verbindungsmolekül (Linker) aus partiell zueinander komplementären einzelsträngigen Oligodesoxynukleotiden hergestellt wird, deren Sequenzen so gewählt sind, dass sie untereinander zu einem oder mehreren nicht-kodierenden Doppelsträngen hybridisieren, wobei gegebenenfalls zwischen diesen Doppelsträngen einzelsträngige Bereiche verbleiben und darüber hinaus kohäsive Enden zur Hybridisierung von Expressionskassetten verbleiben.

9. Verfahren nach Anspruch 7, wobei der Schutz vor Exonukleaseabbau durch eine kurze haarnadelförmige Schleife aus Nukleotiden erreicht wird, die die beiden Stränge des linearen DNA-Doppelstranges kovalent miteinander verknüpft.

10. Verfahren nach Anspruch 9, wobei die haarnadelförmigen Schleifen aus Nukleotiden mit einem oder mehreren Peptiden an einem oder mehreren kovalent modifizerten Nukleosidresten der Nukleoside der Haarnadelschleife kovalent verknüpft sind.

11. Verzweigtes Verbindungsmolekül (Linker) zur Verknüpfung von wenigstens zwei DNA-Expressionskassetten zur Herstellung eines DNA-Expressionskonstruktes zur multiplen Genexpression, bestehend aus partiell zueinander komplementären einzelsträngigen Oligodesoxynukleotiden, wobei diese untereinander zu einem oder mehreren nicht-kodierenden Doppelsträngen mit kohäsiven Enden zur Hybridisierung von Expressionskassetten hybridisieren und die Doppelstränge über Holliday-Junctions miteinander verbunden sind.

12. DNA-Expressionskonstrukts gemäß den Ansprüchen 1 bis 6 zur Verwendung in einem Verfahren zur Behandlung von Tumorerkrankungen.

13. Kit zur Herstellung von DNA-Expressionskonstrukten für die multiple Genexpression, welcher Mittel zur Hybridisierung und Ligation von Oligodesoxynukleotiden und Oligodesoxynukleotide aufweist, deren Sequenzen so gewählt sind, dass sie zu verzweigten Linkern mit einem oder mehreren nicht-kodierenden Doppelsträngen mit kohäsiven Enden zur Hybridisierung von Expressionskassetten hybridisieren und die Doppelstränge über Holliday-Junctions miteinander verbunden sind.

## Claims

1. DNA expression construct for multiple gene expression,
- comprising double stranded regions containing at least two linear expression cassettes, each of which comprises at least a promoter sequence and a coding sequence,
- and where said double stranded regions are connected to each other by a branched linker via holiday junctions,
- and where said expression cassettes are protected against exonuclease degradation on those ends that are not connected to other expression cassettes by DNA single strands or non-coding DNA double strands.

2. DNA expression construct for multiple gene expression according to claim 1, where the expression cassettes encode identical or different genes.

3. DNA expression construct for multiple gene expression according to claim 1, where the protection from exonuclease degradation is achieved by a short single stranded region of three to eight deoxyribonucleotides, which links the two strands of the linear double stranded region to each other covalently.

4. DNA expression construct for multiple gene expression according to claim 3, where said construct is attached covalently to one or several peptides in said short single stranded region.

5. DNA expression construct for multiple gene expression according to claim 4, where said peptide comprises between 3 and 30 amino acids, at least half of which are basic amino acids selected from the group of arginine and lysine.

6. DNA expression construct for multiple gene expression according to claim 4 or 5, where the peptide comprises the amino acid sequence PKKKRKV (Proline-Lysine-Lysine-Lysine-Arginine-Lysine-Valine).

7. Method for the synthesis of a DNA expression construct according to one of claims 1 to 6,
a) where single stranded oligodeoxynucleotides that are partially complimentary to each other are hybridized to give a branched linker molecule,
b) where hybridization is incomplete in such a way that two or more cohesive ends with similar or different base sequence result and
c) where subsequently expression cassettes that are independent of each other and consist of linear, double stranded DNA strands comprising at least a promoter sequence and a coding sequence, are ligated via Holliday junctions to complementary overhangs on said cohesive ends of said linker molecule, and
d) where the double stranded expression cassettes are protected against exonuclease degradation on their ends,
e) where the DNA expression construct thus obtained may be expanded by further branched linker molecules for the conjugation of expression cassettes via Holliday junctions by repeating steps a) to c).

8. Method according to claim 7, where the branched linker molecule is made from single stranded oligodeoxynucleotides that are partially complementary to each other, and the sequences of which are selected in such a way that they hybridize to each other to give one or several non-coding double strands, where between these double strands single strands remain and furthermore, cohesive ends for hybridization of expression cassettes remain.

9. Method according to claim 7, where the protection from exonuclease degradation is achieved by a short hairpin-shaped loop of nucleotides that covalently attaches the two strands of the linear DNA double strand to each other.

10. Method according to claim 9, where one or several peptides are covalently attached to one or several modified nucleoside residues of the nucleoside residues in the hairpin-shaped loop of nucleotides.

11. Branched linker molecule (linker) for the linking of at least two DNA expression cassettes for the production of a DNA expression construct for multiple gene expression, comprising partially complementary to each other, single stranded oligodeoxynucleotides, whereas they hybridize to each other to one or several non-coding double strands with cohesive ends for the hybridization of expression cassettes and the double strands are linked via Holliday junctions to each other.

12. DNA expression construct according to the claims 1 to 6 for the use in a method for the treatment of tumour diseases.

13. Kit for the production of DNA expression constructs for multiple gene expression, comprising means for hybridization and ligation of oligodeoxynucleotides and oligodeoxynuclotide, which sequences are chosen in a way, that they hybridize to branched linker molecules with one or several non-coding double strands with cohesive ends for the hybridization of expression cassettes and the double strands are linked via Holliday junctions to each other.

## Revendications

1. Produit recombiné d'expression d'ADN pour l'expression génique multiple,
- constitué par des domaines doubles brins qui contiennent au moins deux cassettes d'expression qui présentent respectivement au moins une séquence de promoteur et une séquence codante,
- et dans lequel ces domaines doubles brins sont reliés les uns aux autres via un lieur ramifié par des jonctions de Holliday,
- et les cassettes d'expression, aux extrémités auxquelles elles ne sont pas reliées à d'autres cassettes d'expression via de simples brins d'ADN ou les unes aux autres via des doubles brins d'ADN non codants, étant protégées contre une dégradation par des exonucléases.

2. Produit recombiné d'expression d'ADN pour l'expression génique multiple selon la revendication 1, dans lequel les cassettes d'expression codent pour des gènes identiques ou différents.

3. Produit recombiné d'expression d'ADN pour l'expression génique multiple selon la revendication 1, dans lequel la protection contre une dégradation par des exonucléases est obtenue à l'aide d'un court domaine simple brin constitué par un nombre de trois à huit désoxynucléotides, qui relie l'un à l'autre de manière covalente les deux brins du double brin linéaire.

4. Produit recombiné d'expression d'ADN pour l'expression génique multiple selon la revendication 3, dans lequel le produit en question est relié de manière covalente, dans le court domaine simple brin, à un ou plusieurs peptides.

5. Produit recombiné d'expression d'ADN pour l'expression génique multiple selon la revendication 4, dans lequel le peptide est constitué par un nombre d'acides aminés entre 3 et 30, au moins la moitié de ce nombre représentant des acides aminés basiques choisis parmi le groupe comprenant l'arginine et la lysine.

6. Produit recombiné d'expression d'ADN pour l'expression génique multiple selon la revendication 4 ou 5, dans lequel le peptide présente la séquence d'acides aminés PKKKRKV (proline-lysine-lysine-lysine-arginine-lysine-valine).

7. Procédé pour la préparation d'un produit recombiné d'expression d'ADN selon l'une quelconque des revendications 1 à 6 :
a) dans lequel des oligodésoxynucléotides simples brins manifestant une complémentarité réciproque partielle sont soumis à une hybridation pour obtenir une molécule de liaison ramifiée (lieur) ;
b) dans lequel l'hybridation se déroule de manière incomplète de telle sorte qu'elle donne lieu à deux extrémités cohésives ou plus possédant une séquence de bases identique ou différente ; et
c) dans lequel des cassettes d'expression indépendantes les unes des autres, constituées par des doubles brins d'ADN linéaires, qui sont constitués par au moins une séquence de promoteur et par une séquence codante, sont ensuite liées via des extensions complémentaires aux extrémités cohésives de la molécule de liaison (lieur) à l'aide de jonctions de Holliday ; et
d) dans lequel les cassettes d'expression doubles brins en position terminale sont protégées contre une dégradation par des exonucléases,
e) dans lequel le produit recombiné d'expression d'ADN préparé de la sorte peut être augmenté, le cas échéant, par répétition des étapes opératoires a) à c), d'autres molécules de liaison ramifiées (lieurs) à des fins de conjugaison de cassettes d'expression à l'aide de jonctions de Holliday.

8. Procédé selon la revendication 7, dans lequel la molécule de liaison ramifiée (lieur) est préparée à partir d'oligodésoxynucléotides simples brins manifestant une complémentarité réciproque partielle, dont les séquences sont sélectionnées de telle sorte qu'ils s'hybrident l'un à l'autre pour obtenir un ou plusieurs doubles brins non codants, des domaines simples brins subsistant le cas échéant entre ces doubles brins et des extrémités cohésives subsistant en outre à des fins d'hybridation de cassettes d'expression.

9. Procédé selon la revendication 7, dans lequel on obtient la protection contre une dégradation par des exonucléases à l'aide d'une courte boucle en forme d'épingle à cheveux, constituée par des nucléotides, qui relie l'un à l'autre de manière covalente les deux brins du double brin d'ADN linéaire.

10. Procédé selon la revendication 9, dans lequel les boucles en forme d'épingles à cheveux constituées par des nucléotides comprenant un ou plusieurs peptides sont reliées de manière covalente à un ou plusieurs résidus nucléosidiques soumis à une modification covalente des nucléosides de la boucle en forme d'épingle à cheveux.

11. Molécule de liaison ramifiée (lieur) à des fins de liaison d'au moins deux cassettes d'expression d'ADN pour la préparation d'un produit recombiné d'expression d'ADN destiné à l'expression génique, constituée par des oligodésoxynucléotides simples brins manifestant une complémentarité réciproque partielle, ceux-ci s'hybridant l'un à l'autre pour obtenir un ou plusieurs doubles brins non codants comprenant des extrémités cohésives à des fins d'hybridation de cassettes d'expression et les doubles brins étant reliés les uns aux autres à l'aide de jonctions de Holliday.

12. Produit recombiné d'expression d'ADN selon les revendications 1 à 6, à utiliser dans un procédé pour le traitement de maladies tumorales.

13. Nécessaire pour la préparation de produits recombinés d'expression d'ADN pour l'expression génique multiple, qui présente des agents pour l'hybridation et la ligature d'oligodésoxynucléotides et d'oligodésoxynucléotides dont les séquences sont sélectionnées de telle sorte qu'ils s'hybrident, pour obtenir des lieurs ramifiés comprenant un ou plusieurs doubles brins non codants munis d'extrémités cohésives pour l'hybridation de cassettes d'expression et les doubles brins étant reliés les uns aux autres à l'aide de jonctions de Holliday.


### SEQUENCE LISTING

<110> Mologen AG
<120> DNA-Expressionskonstrukt zur multiplen Genexpression
<130> XI 1052/03
<140> PCT/DE03/02478
   <141> 2003-07-19
<150> DE 102 33 812.4
   <151> 2002-07-19
<160> 18
<170> PatentIn version 3.1
<210> 1
   <211> 36
   <212> DNA
   <213> Artificial
<220>
   <221> misc_feature
   <223> synthetic oligonucleotide J3456-1
<400> 1
   gggaggttca gccgcaatcc tcgcctgcac tctacc 36
<210> 2
   <211> 36
   <212> DNA
   <213> Artificial
<220>
   <221> misc_feature
   <223> synthetic oligonuclotide J3456-2
<400> 2
   cctcggtaga gtgcaggcga tgagcacgag tcttgc 36
<210> 3
   <211> 36
   <212> DNA
   <213> Artificial
<220>
   <221> misc_feature
   <223> synthetic oligonucleotide J3-3
<400> 3
   ctccgcaaga ctcgtgctca ggattgcggc tgaacc 36
<210> 4
   <211> 36
   <212> DNA
   <213> Artificial
<220>
   <221> misc_feature
   <223> synthetic oligonucleotide J456-3
<400> 4
   ctccgcaaga ctcgtgctca ccgaatgcca ccacgc 36
<210> 5
   <211> 36
   <212> DNA
   <213> Artificial
<220>
   <221> misc_feature
   <223> synthetic oligonucleotide J4-4
<400> 5
   ggctgcgtgg tggcattcgg ggattgcggc tgaacc 36
<210> 6
   <211> 36
   <212> DNA
   <213> Artificial
<220>
   <221> misc_feature
   <223> synthetic oligonucleotide J5-4
<400> 6
   ggctgcgtgg tggcattcgg actatggcac acatcc 36
<210> 7
   <211> 36
   <212> DNA
   <213> Artificial
<220>
   <221> misc_feature
   <223> synthetic oligonucleotide J6-4
<400> 7
   ggctgcgtgg tggcattcgg cgtccagctc tgatcc 36
<210> 8
   <211> 36
   <212> DNA
   <213> Artificial
<220>
   <221> misc_feature
   <223> synthetic oligonucleotide J6-5
<400> 8
   gtcgggatca gagctggacg actatggcac acatcc 36
<210> 9
   <211> 36
   <212> DNA
   <213> Artificial
<220>
   <221> misc_feature
   <223> synthetic oligonucleotide J56-5/6
<400> 9
   acgcggatgt gtgccatagt ggattgcggc tgaacc 36
<210> 10
   <211> 36
   <212> DNA
   <213> Artificial
<220>
   <221> misc_feature
   <223> synthetic oligonucleotide J2-2
<400> 10
   cctcggtaga gtgcaggcga ggattgcggc tgaacc 36
<210> 11
   <211> 36
   <212> DNA
   <213> Artificial
<220>
   <221> misc_feature
   <223> synthetic oligonucleotide J2-1D
<400> 11
   agggggttca gccgcaatcc tcgcctgcac tctacc 36
<210> 12
   <211> 36
   <212> DNA
   <213> Artificial
<220>
   <221> misc_feature
   <223> synthetic oligonucleotide J2-2D
<400> 12
   agggggtaga gtgcaggcga ggattgcggc tgaacc 36
<210> 13
   <211> 36
   <212> DNA
   <213> Artificial
<220>
   <221> misc_feature
   <223> synthetic oligonucleotide J2-2U
<400> 13
   gggaggtaga gtgcaggcga ggattgcggc tgaacc 36
<210> 14
   <211> 36
   <212> DNA
   <213> Artificial
<220>
   <221> misc_feature
   <223> synthetic oligonucleotide J2-2U (CTCC)
<400> 14
   ctccggtaga gtgcaggcga ggattgcggc tgaacc 36
<210> 15
   <211> 36
   <212> DNA
   <213> Artificial
<220>
   <221> misc_feature
   <223> synthetic oligonucleotide
<220>
   <221> misc_feature
   <223> synthetic oligonucleotide J2-2D (GGCT)
<400> 15
   ggctggtaga gtgcaggcga ggattgcggc tgaacc 36
<210> 16
   <211> 36
   <212> DNA
   <213> Artificial
<220>
   <221> misc_feature
   <223> synthetic oligonucleotide J3456-2 /ggga
<400> 16
   gggaggtaga gtgcaggcga tgagcacgag tcttgc 36
<210> 17
   <211> 36
   <212> DNA
   <213> Artificial
<220>
   <221> misc_feature
   <223> synthetic oligonucleotide J456-3 /ggga
<400> 17
   gggagcaaga ctcgtgctca ccgaatgcca ccacgc 36
<210> 18
   <211> 7
   <212> PRT
   <213> Simian virus 40
<220>
   <221> MISC_FEATURE
   <223> NLS-peptide
<400> 18
   Pro Lys Lys Lys Arg Lys Val
   1               5